# EUROPEAN PATENT SPECIFICATION

(11) **EP 2 969 035 B1**
(45) Date of publication and mention of the grant of the patent: **29.11.2017**
(21) Application number: 14769375.8
(22) Date of filing: 13.03.2014
(51) Int. Cl.: A61M 16/00, A61M 16/06, A61M 16/08, A61M 16/20

(54) **DUAL PRESSURE SENSOR PATIENT VENTILATOR**
PATIENTENBEATMUNGSVORRICHTUNG MIT ZWEI DRUCKSENSOREN
VENTILATEUR DE PATIENT À DOUBLE CAPTEUR DE PRESSION

(30) Priority: 15.03.2013 US 201313841189
(43) Date of publication of application: 20.01.2016
(73) Proprietor: Breathe Technologies, Inc., Irvine, CA 92618 (US)
(72) Inventor: AHMAD, Samir S., San Diego, California 92130 (US); BALOA WELZIEN, Leonardo Alberto, Lake Forest, California 92630 (US); BRAMBILLA, Enrico, Irvine, California 92604 (US)
(74) Representative: Pallini Gervasi, Diego
(86) International application number: PCT/US2014/026475
(87) International publication number: WO 2014/151804

(56) References cited:
- US-A- 5 813 399
- US-A1- 2004 103 896
- US-A1- 2004 103 896
- US-A1- 2008 135 044
- US-A1- 2008 135 044
- US-A1- 2010 071 697
- US-A1- 2010 078 024
- US-A1- 2010 078 024

## Description

### BACKGROUND

### 1. Field of the Invention

The present disclosure relates generally to the patient ventilation systems, and more particularly, to methods and systems for non-invasive ventilation in which a pressurized flow of breathable gas is provided to a patient utilizing dual pressure sensors at a source and at a ventilation mask.

### 2. Description of the Related Art

The respiration system of the human body provides needed oxygen intake, oxygen/carbon dioxide exchange, and carbon dioxide expulsion functions, each of which involves the lungs. In this regard, the lungs function as a gas-exchanging organ in which inhaled oxygen is passed to the blood, and collected carbon dioxide is passed from the blood to the air. Additionally, the lungs function as a respiratory pump that transports oxygen-rich air into the lungs, and the carbon dioxide-rich air out of the lungs. The breathing center in the brain, central and peripheral nerves, the osseous thorax and the breathing musculature as well as free, stable respiratory paths are necessary for a correct functioning of the respiratory pump.

With patients suffering from certain diseases or other serious medical conditions, there is a constant overload on or exhaustion of the respiratory pump. A typical syndrome is pulmonary emphysema with flat-standing diaphragms without the ability to contract, and the respiratory paths are usually extremely slack and tend to collapse. Consequentially, the patient experiences difficulty with breathing deeply enough and providing the body with needed oxygen while also expelling waste carbon dioxide.

Mechanical ventilators comprise medical devices that either perform or supplement breathing for patients. Early ventilators, such as the "iron lung," created negative pressure around the patient's chest to cause a flow of ambient air through the patient's nose and/or mouth into the lungs. However, the vast majority of contemporary ventilators instead use positive pressure to deliver gas to the patient's lungs via a patient circuit between the ventilator and the patient. The patient circuit typically consists of one or two large bore tubes (e.g., 22mm inner diameter for adults; 15mm inner diameter for pediatrics) that interface to the ventilator on one end and a patient mask on the other end.

Current ventilators are designed to support either "vented" or "leak" circuits, or "non-vented" or "non-leak" circuits. Ventilators using vented circuits are most typically used for less acute clinical requirements, such as the treatment of obstructive sleep apnea or respiratory insufficiency. On the other hand, non-vented circuits in which there are no vent openings in the patient interface, are typically used for critical care applications.

Non-vented or non-leak ventilators may utilize either a single limb or a dual limb patient circuit. As its nomenclature suggests, single limb patient circuits involves gas flow from the ventilator to the patient and patient mask over a single conduit. Single limb patient circuits are typically utilized for less acute clinical requirements such as the treatment of obstructive sleep apnea or respiratory insufficiency. Dual limb circuits involve one conduit carrying gas flow from the ventilator to the patient interface, and another conduit carrying the exhaled gas from the patient's lungs back to the ventilator.

The ventilator applies positive pressure to open the patient's airway to prevent its collapse. In basic implementations, the rate and volume of inhalation and exhalation cycles is set by the ventilator without regard to the patient's spontaneous breathing cycle. However, to the extent the patient is capable of spontaneous respiration, any positive or negative pressure applied by the ventilator in opposition thereto may cause discomfort. Notwithstanding the clinician's best efforts to prescribe a ventilation flow rate that minimizes such extraneous pressure augmentation while ensuring the proper splinting of the airway during inspiration, the patient may be subject to higher pressures than needed throughout the breathing cycle. US2010/078024 A1, US2004/103896 A1, US2010/071697 and US5813399 are examples of breathing assistance devices. Accordingly, there is a need in the art for an improved system for ventilation including dual pressure sensors at a source and on a ventilation mask to control an exhalation valve.

### BRIEF SUMMARY

According to various embodiments of the present disclosure, a ventilation apparatus is contemplated. The invention is defined by the appended claim 1. The apparatus may include an inlet port that is connectible to an oxygen source with pressurized oxygen enriched gas. Additionally, there may be an outlet port that is connectible to a patient interface over a gas delivery conduit. The patient interface may be configured for fitment on a patient respiratory passageway. The apparatus may further include a valve with an input and an output, and the input may be in pneumatic communication with the inlet port and the output may be in pneumatic communication with the outlet port. There may be a first pressure sensor that measures a patient interface pressure. The patient interface may be connectible to the first pressure sensor over a pressure sensor line. The apparatus may also include a second pressure sensor that measures a valve output pressure. There may be a controller in communication with the first pressure sensor, the second pressure sensor, and the flow sensor. A patient inspiratory phase and a patient expiratory phase may be detectable by the controller based upon a combination of measurements of the first pressure sensor and the second pressure sensor to regulate the valve to selectively deliver the pressurized oxygen enriched gas to the patient interface. The present disclosure will be best understood by reference to the following detailed description when read in conjunction with the accompanying drawings.

### BRIEF DESCRIPTION OF THE DRAWINGS

These and other features and advantages of the various embodiments disclosed herein will be better understood with respect to the following description and drawings, in which like numbers refer to like parts throughout, and in which:
FIG. 1 is a block diagram showing the various components of a ventilator apparatus in accordance with various embodiments of the present disclosure including a ventilation unit, a patient ventilation interface, gas passage conduits, and oxygen sources;
FIG. 2 is a block diagram illustrating the electrical components of the ventilation unit;
FIG. 3 is a pressure diagram graphically illustrating the pressure cycles at the oxygen source valve and the patient ventilation interface over a typical breathing sequence including inspiratory phases and expiratory phases;
FIG. 4 is a pressure diagram graphically illustrating the pressure cycle differences (ΔP) at the oxygen source valve the patient ventilation interface over the breathing sequence;
FIG. 5 is a pressure diagram graphically illustrating the pressure difference (ΔP) at the oxygen source valve and the patient ventilation interface showing a leak constant;
FIG. 6 is a control loop block diagram depicting pressure sensor variables relative to the oxygen source valve and the patient ventilation interface as inputs to control devices;
FIG. 7 is a control loop block diagram depicting a patient ventilation interface pressure control;
FIG. 8 is a control loop block diagram depicting flow sensor and pressure sensor variables relative to the oxygen source valve and the patient ventilation interface as inputs to control devices;
FIG. 9 is a flowchart illustrating the processing steps of the control loop shown in FIG. 6;
FIG. 10 is a block flow diagram showing the interrelated components of the ventilation unit.

Common reference numerals are used throughout the drawings and the detailed description to indicate the same elements.

### DETAILED DESCRIPTION

The detailed description set forth below in connection with the appended drawings is intended as a description of the several presently contemplated examples of a system for continuous patient ventilation. The system delivers breathing gas to a patient for respiratory assistance and implements various methods for the selective pressure augmentation throughout the breathing cycle. This description is not intended to represent the only form in which the disclosed invention may be developed or utilized. The description sets forth the functions and features in connection with the illustrated embodiments. It is to be understood, however, that the same or equivalent functions may be accomplished by different embodiments that are also intended to be encompassed within the scope of the present disclosure. It is further understood that the use of relational terms such as first and second and the like are used solely to distinguish one from another entity without necessarily requiring or implying any actual such relationship or order between such entities.

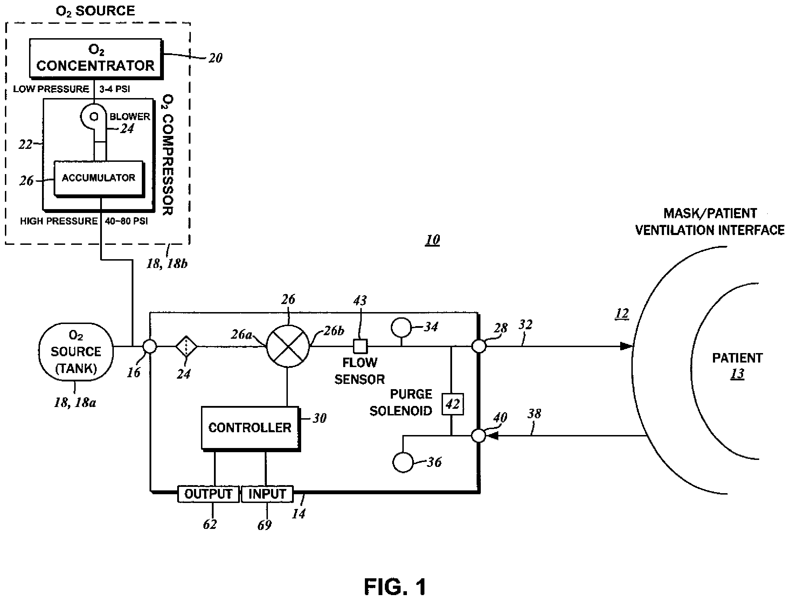

With reference to the block diagram of FIG. 1, one embodiment of the present disclosure contemplates a ventilation system 10 generally comprised of a patient ventilation interface 12 and a ventilation unit 14. The patient ventilation interface 12 may include such devices as a full-face mask or a nasal mask that can be placed in direct gas flow communication with the upper respiratory tract of the patient, i.e., the nasal cavity and the oral cavity. One embodiment of the ventilation system 10 may utilize a nasal mask. It will be appreciated that other apparatuses that so interface the respiratory system of the patient to the ventilation unit 14 may be substituted without departing from the scope of the present disclosure, so long as certain features noted below are incorporated.

Generally, the ventilation unit 14 generates a flow of breathing gas that is delivered to the patient via the patient ventilation interface 12. The breathing gas may be a combination of ambient air enriched with oxygen, or any other suitable mixture of gas appropriate for treating the patient. The ventilation unit 14 includes a first inlet port 16, through which oxygen enriched gas is provided by an oxygen source 18. A first type of oxygen source is a tank 18a, which stores compressed, oxygen enriched gas. Alternatively, there may be a second type, which is comprised of a oxygen concentrator device 20 that intakes ambient air and outputs a lower pressure oxygen-enriched gas to an oxygen compressor 22. According to one embodiment, the oxygen concentrator 22 generates an output pressure of approximately 0.2 to 0.27 Bar (3 to 4 psi). In further detail, the oxygen compressor 22 includes a blower device 24 that increases the pressure of gas flow ported from the oxygen concentrator 20 for storage in an accumulator 24. Output from the accumulator 24 is regulated at a comparatively higher pressure of approximately 2.75 to 5.51 Bar (40 to 80 psi). The first inlet port 16 of the ventilation unit 14 is in communication with an inlet filter 18 that removes particulates and other contaminants from the breathing gas that is ultimately delivered to the patient.

The high pressure originating from either of the oxygen sources 18a, 18b is regulated by a valve 26. There is a valve inlet port 26a in gas flow communication with the inlet filter 18, and a valve outlet port 26b that is in gas flow communication with an outlet port 28 of the ventilation unit 14. As will be described in further detail below, the position of the valve 26 is selectively adjusted to port a desired volume/pressure of gas from the oxygen sources 18 to the patient 13. It will be recognized that any suitable valve 26 capable of regulating gas flow and pressure for ventilating a patient in accordance with the present disclosure may be utilized. The valve 26 may be driven electrically, pneumatically, or any other suitable motive modalities. The actuation of the valve 26 is governed by a programmable controller 30 that implements the various methods of patient ventilation contemplated by the present disclosure, as will be described in further detail below.

The flow of breathing gas that is ported through the valve 26 is passed through the outlet port 28 to a gas delivery conduit 30 that is coupled to the aforementioned mask or patient ventilation interface 12. The gas delivery conduit 30 is understood to be a plastic tube having a predetermined inner diameter such as 22mm or smaller, though any other conduit of suitable material and construction may be utilized. A pressure difference is generated between the patient ventilation interface 12 and the output of the valve 26, i.e., the valve outlet 26a depending on the state of respiration of the patient 13.

In order to ascertain such pressure differentials, the presently contemplated ventilation system 10 includes dual pressure sensors, including a valve pressure sensor 34 and a patient interface pressure sensor 36. The valve pressure sensor 34 is disposed within the ventilation unit 14, and monitors the pressure at the valve outlet port 26b. The patient interface pressure sensor 36 is also physically disposed within the ventilation unit 14, but is in direct gas flow communication with the patient ventilation interface 12 over a pressure sensor line 38 that is connected to a sensor inlet port 40. When the ventilation unit 14 is operating, gas pressure within the pressure sensor line 38 as well as the gas conduit 32 may be connected to deliver a purge flow to clear the pressure sensor line 38. This can be done through a purge solenoid 42 connected to both. The purge can be continuous or intermittent according to the patient's breathing phase or pressure difference between the valve pressure and the patient interface pressure.

With the reduced diameter of the gas delivery conduit 30, particularly in relation to a larger diameter sections of the gas flow passageway through the valve 26, there are increased flow rates at lower pressures at the patient ventilation interface 12. As will be recognized by those having ordinary skill in the art, a Venturi effect is exhibited.

In addition to measuring pressure differentials at the patient ventilation interface 12 and the valve output 26b, flow measurements of the breathing gas actually output from the valve 12 are utilized. To this end, the ventilation unit 14 includes a flow sensor 43 that is in-line with the valve 12 and the outlet port 28.

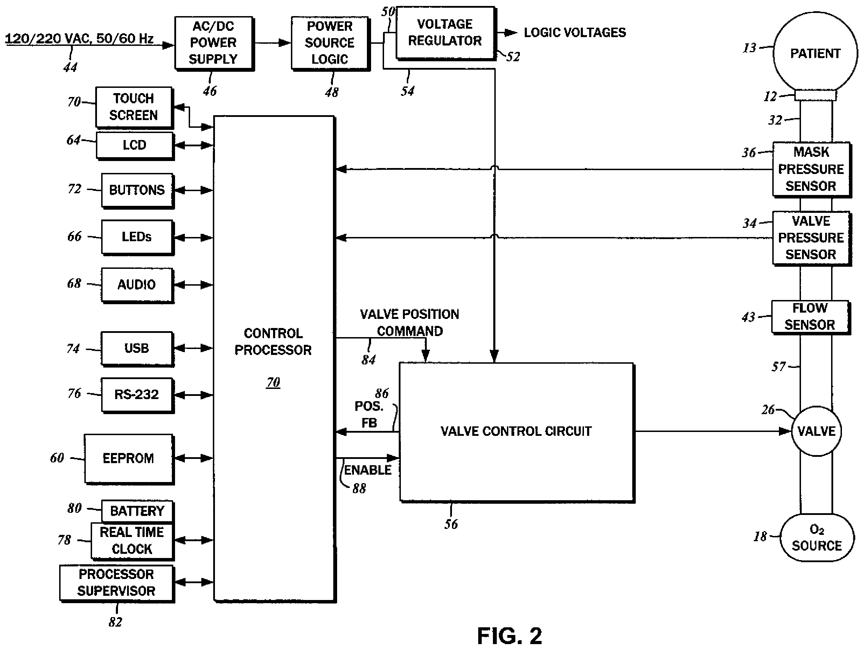

The block diagram of FIG. 2 illustrates the various electrical components of one typical embodiment of the ventilation unit 14. Power for the ventilation unit 14 may be provided from a conventional household electricity supply of either 120V or 220V alternating current (AC), at 50Hz or 60Hz. The block diagram denotes this supply as a power source 44. A power supply 46 is connected to the power source 44, and as will be recognized by those having ordinary skill in the art, the power signal is variously rectified, filtered, and stepped down to a direct current (DC) voltage. In accordance with one embodiment of the present disclosure, the DC voltage source is 24 V. Some components of the ventilation unit 14 may utilize higher DC voltages than control logic devices, and so the power supply 46 is connected to a power source logic 48. A first output 50 of the power source logic 48 is connected to an integrated circuit voltage regulator 52 that steps down the DC voltage to the logic device level of 5V. A second output 54 of the power source logic 48 is the existing high DC voltage directly from the power supply 46, and is connected to a valve control circuit 56.

The continuous source of oxygen enriched breathing gas is provided by the oxygen source 18 as discussed above, and the output to the patient ventilation interface 12 and eventually to the patient 13 is regulated by the valve 26. The specific position of the valve 26 needed for the determined volume and pressure of breathing gas is set by the valve control circuit 56, and those having ordinary skill in the art will recognize implementations of valve actuators that are suitable for the contemplated application. From the opening of the valve 26, breathing gas flows through an internal conduit 57 that is in-line with the flow sensor 43. The internal conduit 57 is coupled to the aforementioned outlet port 24 of the ventilation unit 14, which in turn is coupled to the gas conduit 32. As described above, the valve pressure sensor 34 and the patient interface pressure sensor 36 are connected to the pneumatic circuit between the valve 26 and the patient 13.

The controller 30 and its functionality may be implemented with a programmable integrated circuit device such as a microcontroller or control processor 58. Broadly, the control processor 58 receives certain inputs, and based upon those inputs, generates certain outputs. The specific operations that are performed on the inputs may be programmed as instructions that are executed by the control processor 58. In this regard, the control processor 58 may include an arithmetic/logic unit (ALU), various registers, and input/output ports. Although external memory such as EEPROM (electrically erasable/programmable read only memory) 60 may be connected to the control processor 58 for permanent storage and retrieval of program instructions, there may also be an internal random access memory (RAM). One embodiment contemplates the use of an Intel 8081 instruction set/architecture, though any other suitable instruction set or processor architecture may be substituted. As indicated above, the control processor 58 is powered by a low voltage DC supply from the voltage regulator 52.

In order to set the operational parameters of the ventilation unit 14, and to initiate or terminate certain functions, a graphical user interface is provided. Such graphical user interface may generated on an output device 62, which may be of a liquid crystal (LCD) type 64. Any graphic may be shown on the output device, though for more specific indicators, a simple light emitting diode (LED) device 66 may be utilized. It will be recognized that alarm conditions, power status, and the like may be indicated with the LED device 66. Audible outputs may also be produced with audio transducers 68 that are likewise connected to the control processor 58. Among the contemplated outputs that may be generated on the audio transducer 68 include simple beeps and alarms, as well as sophisticated voice prompts that provide information and instructions.

An operator may interact with the graphical user interface through different input devices 69 such as a touch screen interface 70 that is overlaid on the LCD screen 64. It will be recognized that various graphic elements may be generated on the underlying screen 64, with touch inputs/interactions corresponding in position to those graphic elements being evaluated as a selection or activation of the same. Various touch screen interfaces, some of which may be directly integrated with the screen 64, are known in the art. Besides touch screen inputs, buttons 72 may also be connected to the control processor 58 for similarly receiving user inputs. It is understood that the audio transducer 68 may also accept sound input in the form of voice commands, the processing of which is performed may be performed by the control processor 58.

Several modalities for connecting to and communicating with other data processing devices such as general-purpose computers are also contemplated. Accordingly, the control processor 58 may be connected to a universal serial bus (USB) controller 74. For more basic communications, there may be a serial RS-232 transceiver 76. Through these data communications modalities, the configuration options of the ventilation unit 14 may be set, operating profiles may be downloaded, and so forth. Notwithstanding the specific reference to USB and RS-232 communications modalities, any other communications modality including wireless systems may be substituted without departing from the present disclosure.

The functions of the ventilation unit 14 depend on proper synchronization, and so the control processor 58 is connected to a real time clock 78 that maintains a common clock cycle. Although a primary feature of the real time clock 78 is to maintain synchrony at a processor cycle level, longer term time data is also maintained. In order to retain such time data, the real time clock 78 may be powered independently of the primary power source 46, and there is accordingly a battery backup 80. Under heavy processing loads or unexpected program conditions, the control processor 58 may become unable to execute critical programmed steps in real-time. Thus, the control processor 58 may include a processor supervisor 82 that invokes a program execution break upon detecting such conditions. Typically, this is implemented as a step of clearing a memory variable periodically, and when that step is unable to take place because instruction execution is frozen or otherwise delayed, the processor supervisor 82 may cause a predetermined routine to be executed.

As mentioned above, the valve 26 is driven by the valve control circuit 56, which generates different outputs depending on signals received from the control processor 58. The signal to drive the valve 26 is generated on a valve position command line 84. To confirm that the position of the valve 26 is actually where it has been commanded to be, the valve control circuit 56 provides feedback via a valve position feedback line 86. Furthermore, as detailed below, pressure readings at the patient 13 is utilized to reach control decisions, so the patient interface pressure sensor 36 is connected to the control processor 70. The valve control circuit 56 is activated and deactivated via an enable line 88.

As referenced herein, the terms patient ventilation interface 12 and patient mask are utilized interchangeably. It will be recognized that the patient mask is a specific kind of patient ventilation interface, and as explained briefly above, other types of ventilation interfaces may be utilized. Along these lines, reference to such terms as mask pressure, valve pressure, or the use of the term mask or valve to modify any other term is for purposes of convenience only and not of limitation. For instance, mask pressure is understood to refer to the pressure in the patient ventilation interface 12, while valve pressure refers to the pressure at the output of the valve 26.

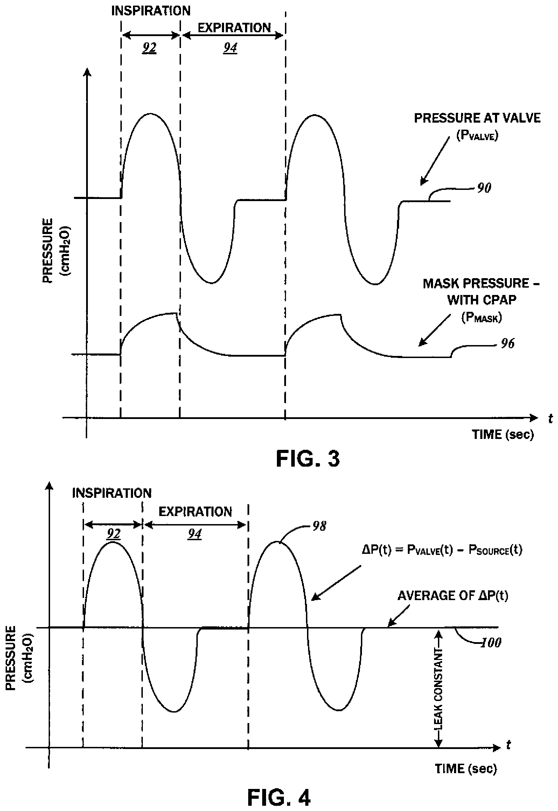

Referring now to the pressure diagram of FIG. 3, the typical operating pressures at the valve 26, and at the patient ventilation interface 12 at different points in the breathing cycle is illustrated. More particularly, a first plot 90 illustrates the pressure cycle at the valve 26 as measured by the valve pressure sensor 34, and is characterized by an inspiration region 92 and an expiration region 94. As will be appreciated, the pressure at the valve 26 increases during inspiration, and decreases during expiration. Henceforth, the first plot 90 and the measurement represented thereby will be referred to as PValve, with the pressure value at any particular time t being referred to as PValve(t). This pressure is given in terms of cmH2O, as are the other pressure measurements discussed herein.

A second plot 96 shows the operating pressure cycle at the patient ventilation interface 12 as measured by the pressure sensor 36. The second plot and the measurements represented thereby will be referred to as PMask, with the pressure value at any particular time t being referred to as PMask(t). As shown in the second plot 96, PMask increases over the inspiration region 92, and decreases over the expiration region. Furthermore, the pressure at the valve 26 PValve is significantly higher than the pressure at the patient ventilation interface 12 PMask.

Patient leak indication is also contemplated for the presently disclosed ventilation system 10. As shown in the graph of FIG. 4, a first plot 98 is representative of the difference between PValve and PMask over a time period at a given instant, and is also referred to as ΔP. The first plot 98 can also be characterized by the inspiration region 92 and an expiration region 94. The average of the pressure difference ΔP is represented as a leak constant 104. It will be recognized that the larger the value of the leak constant 54, the greater the leakage.

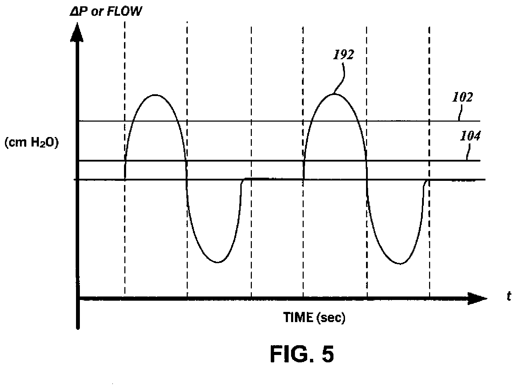

Referring now the graph of FIG. 5, the determination of the patient trigger and cycle states will now be considered. It is understood that cycling and trigger states are based on the patient's breathing cycle, and so the present disclosure contemplates a modality by which the inspiration phase and the expiration phase can be ascertained. A plot 192 represents the pressure difference ΔP between the mask pressure PMask and the valve pressure PValve over a time period. Alternatively, the plot 192 is also representative of the flow rate Q as measured by the flow sensor 43.

In accordance with the invention, a trigger limit 102 is set or otherwise computed as an average of ΔP and/or Q. More particularly, the trigger limit 102 at time (t) may be the average ΔP or flow also at time (t) plus a predetermined trigger constant, which may be set by the clinician or the patient. If the pressure difference ΔP and/or flow Q at (t) is greater than the trigger limit at the same time (t), the patient is considered to be in the inspiration phase.

A cycle limit 104 is also set, and is understood to be a function of the peak value of ΔP and/or Q. In further detail, the cycle limit at time (t) is the maximum ΔP and/or Q at time (t) multiplied by a cycle constant. The cycle constant can also be set by the clinician or the patient, or otherwise computed as a function of ΔP(t). If the measured ΔP is less than the set cycle limit, then the patient is determined to be in the expiration phase.

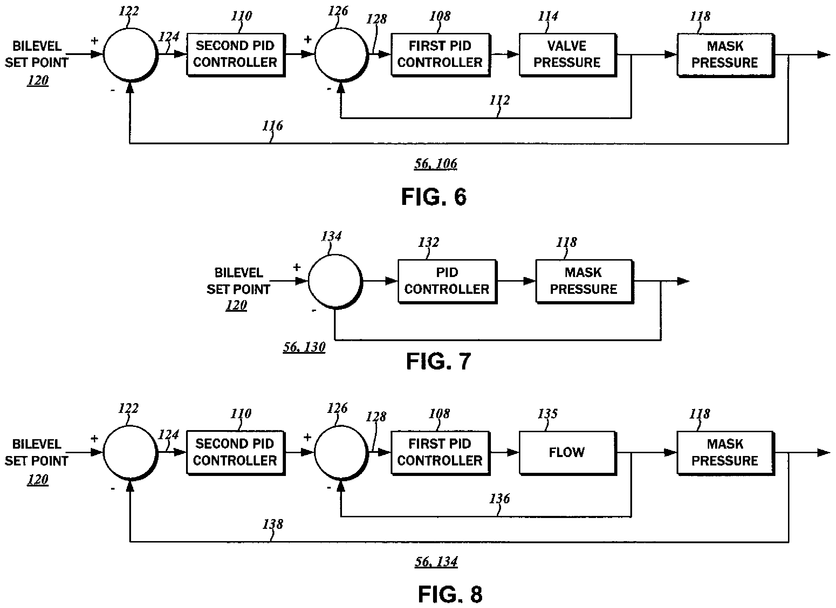

The specific pressure that is to be delivered to the patient 13 from the oxygen source 18 as regulated by the valve 26 is set by the programmable pressure controller 26, and FIG. 6, FIG. 7, and FIG. 8 are control loop block diagrams thereof illustrating the application of different variables. Several implementations of the presently disclosed ventilation system 10 involve bi-level positive airway pressure therapy. A first positive airway pressure is delivered to the patient 13 during the inspiratory phase as detected in accordance with the trigger limit, and a second, lower positive airway pressure is delivered during the expiratory phase as detected in accordance with the cycle limit as discussed above.

Referring specifically to the control loop block diagram of FIG. 6, additional details pertaining to the control functions of the valve 26, and specifically a closed loop control circuit 106 utilizing the pressure differentials between the valve 26 and the patient ventilation interface 12, will now be considered.

Generally, the closed loop control circuit 106 includes a first PID controller 108, and a second PID controller 110, both of which act upon the valve 26 to effectuate pressure changes within the patient circuit. There is a first or inner control loop 112 that is driven by the first PID controller 108 to modulate valve pressure 114, as well as a second or outer control loop 116. Together with the first PID controller 108 and the second PID controller 110, mask pressure 118 is modulated. The inner control loop 112 and the outer control loop 116 are inter-related and together define the closed loop control circuit 106.

One objective of the closed loop control circuit 106 is to operate the valve 26 to the extent necessary to achieve a predetermined pressure sufficient to meet the inspiratory and expiratory pressure demands of the patient and the pressure losses in the ventilation system 14. A desired pressure, which is the preset bi-level positive airway pressure as input by the clinician, is represented by an input value 120 that is provided to a first summing point 122. The pressure at the patient mask, i.e., mask pressure 117, is measured by pressure sensor 36 as discussed above, and also input to the first summing point 122. An output signal 124 corresponding to the summed pressures of the input value 120 and the mask pressure 118 is passed to the second PID controller 110. The output signal 124 is processed by the second PID controller 110, and this processed signal is output to the valve control circuit 56 to partially regulate the valve 26 in response.

The output from the second PID controller 110 is input to a second summing point 126, which also adds the pressure value at the valve, i.e., the valve pressure 114. Another output signal 128 corresponding to these summed pressures is passed to the first PID controller 108, which again is processed and output to the valve control circuit 56 to regulate the valve 26. The subsequent valve pressure 114 measurement is again fed back to the second summing point 126, at which point the inner loop continues.

The first PID controller 108 is thus part of a closed loop control over a valve pressure sensor 34, with the output thereof being the current set point for the valve 26. Furthermore, the second PID controller 110 minimizes the error between the mask pressure and the Bi-level set level 120. The output of the second PID controller 110 is the set pressure for the first PID controller 108. The respective gains of the first PID controller 108 and the second PID controller 110 may be scheduled according to the patient breathing phase and/or the Bi-level set point 120. During expiration, the controller 30 can be reconfigured to control the position of the valve 26, valve pressure, flow, either alone or to different set targets. Upon detecting an inspiration phase, the bi-level set point 120 can be set to a different value than during expiration.

FIG. 7 illustrates a more general closed loop control circuit 130 that regulates the mask pressure 118 with a PID controller 132. The measured mask pressure 118 is provided to a summing point 134 and summed with the bi-level set point 120.

The control loop block diagram of FIG. 8 likewise illustrates control functions of the valve 26 as implemented in a closed loop control circuit 134 utilizing flow measurements through the valve 26 as measured by the flow sensor 43. Like the above-described pressure differential based control system, there is the first PID controller 108 and the second PID controller 110, both of which act upon the valve 26 to effectuate pressure changes within the patient circuit. A first or inner control loop 136 is driven by the first PID controller 108 to modulate flow 135, as well as a second or outer control loop 138. Together with the first PID controller 108 and the second PID controller 110, mask pressure 118 is modulated. The inner control loop 136 and the outer control loop 138 are inter-related and together define the closed loop control circuit 134.

Again, the closed loop control circuit 134 is understood to operate the valve 26 to the extent necessary to achieve a predetermined pressure sufficient to meet the inspiratory and expiratory pressure demands of the patient and the pressure losses in the ventilation system 14. A desired pressure, which is the preset bi-level positive airway pressure as input by the clinician, is represented by an input value 120 that is provided to the first summing point 122. The mask pressure 118 is also input to the first summing point 122. An output signal 124 corresponding to the summed pressures of the input value 120 and the mask pressure 118 is passed to the second PID controller 110. The processed output signal 124 is output to the valve control circuit 56 to partially regulate the valve 26 in response.

The output from the second PID controller 110 is input to the second summing point 126, which is summed with the flow 135. Another output signal 128 corresponding to these summed values is passed to the first PID controller 108, which again is processed and output to the valve control circuit 56 to regulate the valve 26. The subsequent flow measurement is again fed back to the second summing point 126, at which point the inner loop 136 continues.

The first PID controller 108 is part of a closed loop control over the flow sensor 43, with the output thereof being the current set point for the valve 26. Furthermore, the second PID controller 110 minimizes the error between the mask pressure and the Bi-level set level 120. The output of the second PID controller 110 is the set pressure for the first PID controller 108. The respective gains of the first PID controller 108 and the second PID controller 110 may be scheduled according to the patient breathing phase and/or the Bi-level set point 120. During expiration, the controller 30 can be reconfigured to control the position of the valve 26, valve pressure, flow, either alone or to different set targets. Upon detecting an inspiration phase, the bi-level set point 120 can be set to a different value than during expiration.

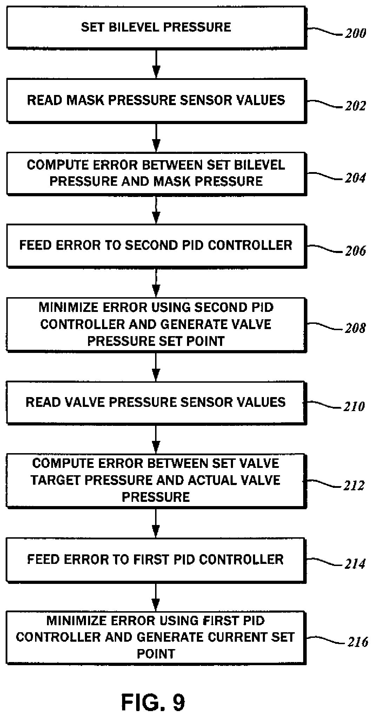

With reference to the flowchart of FIG. 9, the steps involved in the control loop circuits 106 and 134 will be described. In a step 200, the bi-level set point 120 is set. The mask pressure 118 is then read in a step 202, and the error between the set bi-level set point pressure 120 and the mask pressure 118 is computed in a step 204, as described above in relation to the second summing point 122 In a step 206, the computed error is fed to the second PID controller 110. The valve 26 is adjusted by the second PID controller 110 in a step 208 to minimize the error between the bi-level set point 120 and the mask pressure 118. At this point, the valve pressure set point is generated. Per the inner control loop 112, the valve pressure 114 is received in a step 210, and an error between such value and the set valve pressure set point from the second PID controller 110 is computed in a step 212. The amount of error is then fed to the first PID controller 108 in accordance with a step 214. The error is minimized by further adjusting the valve 26 in a step 216, thereby generating the current set point.

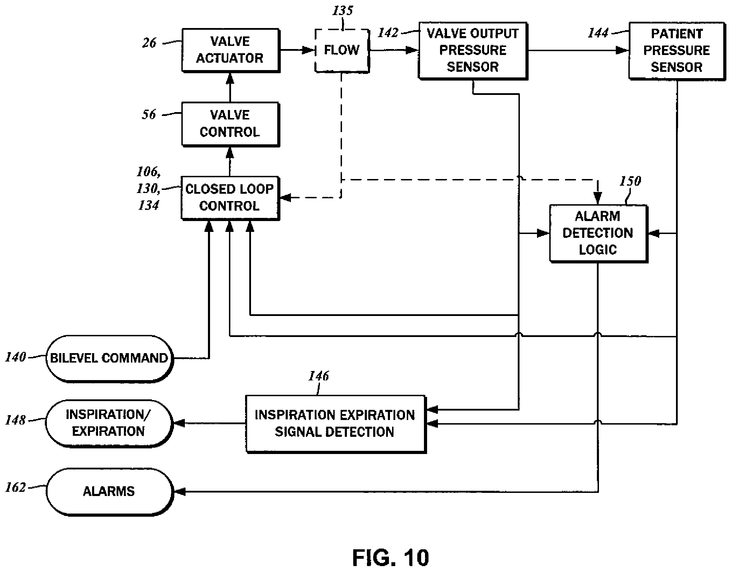

Referring now to the diagram of FIG. 10, the operational sequence of the ventilation system 10 will be considered. As indicated above, the valve 26 is actuated by the valve control circuit 56. In particular, the position of the valve 26 is electrically or pneumatically moved to the defined position that achieves the needed flow rates and pressure. The specific sequence and manner in which this occurs is governed by the closed loop control circuits 102, 130, and 134 that is implemented by the controller 56. One of the inputs to the closed loop control circuit 156 is a pressure command 140, or the therapeutic pressure that is set by a clinician.

Actuating the valve 26 results in a change in pressure at the valve 25, as sensed in a valve pressure sensor block 142. Furthermore, pressure readings are also made at the patient ventilation interface 12, or a mask pressure sensor block 144. Optionally, there is also a flow sensor block 145. These readings are inputs to the closed loop control circuit 56. Additionally, the readings from the valve pressure sensor block 142, the mask pressure sensor block 144 and the flow sensor block 145 are utilized in a breathing cycle state detector block 146. As mentioned above, the pressure difference (P) can be utilized to determine whether the patient 13 is in an expiration (exhalation) state or an inspiration (inhalation state). The breathing cycle state detector 146 so utilizes the pressure measurements and generates a breathing cycle state output 148.

Different alarm conditions may be evaluated based in part on the pressure difference between the measurements from the valve pressure sensor block 42, the mask pressure sensor block 144, and optionally, the flow sensor block 145. These values are passed to an alarm detection logic block 150 that can trigger an alarm 152.

The particulars shown herein are by way of example and for purposes of illustrative discussion of the embodiments of the present disclosure only and are presented in the cause of providing what is believed to be the most useful and readily understood description of the principles and conceptual aspects. In this regard, no attempt is made to show details of the present invention with more particularity than is necessary, the description taken with the drawings making apparent to those skilled in the art how the several forms of the present invention may be embodied in practice.

## Claims

1. A ventilation apparatus comprising:
an inlet port (16) connectible to an oxygen source (18, 18a, 18b) with pressurized oxygen enriched gas;
an outlet port (28) connectible over a gas delivery conduit (30) to a patient interface (12) configured for fitment on a patient respiratory passageway;
a valve (26) with an input in pneumatic communication with the inlet port (16) and an output in pneumatic communication with the outlet port (28);
a first pressure sensor (36) measuring a patient interface pressure, the patient interface (12) being connectible to the first pressure sensor (36) over a pressure sensor line (38);
a second pressure sensor (34) measuring a valve output pressure; and
a controller in communication with the first pressure sensor (36) and the second pressure sensor (34) **characterised in that** the controller computes an average of a pressure difference ΔP between the valve output pressure and the patient interface pressure and detects a patient inspiratory phase based upon a comparison between the pressure difference ΔP and the average..

2. The ventilation apparatus of Claim 1, wherein a diameter of the valve (26) in a fully open position is greater than a diameter of the gas delivery conduit (30).

3. The apparatus of Claim 1, further comprising:
a flow sensor (43) measuring a gas flow rate and in-line with the output of the valve (26) and the outlet port (28);
wherein the controller detects the patient inspiratory phase and the patient expiratory phase based upon a combination of measurements of the first pressure sensor (36), the second pressure sensor (34), and the flow sensor (43).

4. The apparatus of Claim 3, wherein the controller regulates the delivery of the pressurized oxygen enriched gas to the patient interface (12) according to a trigger limit and a cycle limit reached by the gas flow rate; preferably
wherein an average gas flow rate over one or more patient breathing cycles defines a leak constant; optionally
wherein the trigger limit is defined by the leak constant added to a trigger constant.

5. The apparatus of Claim 4, wherein the cycle limit is defined by a cycle constant fraction of a maximum flow rate.

6. The apparatus of Claim 3, wherein the controller opens the valve (26) to induce a flow of the pressurized oxygen enriched gas to the patient interface (12) and closes the valve (26) to reduce the flow.

7. The apparatus of Claim 3, wherein the controller includes a first proportional-integral-derivative (PID) controller (108) and a second PID controller (110), the first PID controller (108) being part of a first control loop over the gas flow rate, and the second PID controller (110), together with the first control loop, being part of a second control loop for minimizing error between the patient interface pressure and a set pressure.

8. The apparatus of Claim 1, wherein the controller opens the valve (26) to induce a pressure differential at the patient ventilation interface (12) and closes the valve (26) to reduce the pressure differential.

9. The apparatus of Claim 1, wherein the controller regulates the delivery of the pressurized oxygen enriched gas to the patient interface (12) according to a trigger limit and a cycle limit reached by the pressure differentials between the patient interface pressure and the valve output pressure; preferably wherein an average pressure differential over one or more patient breathing cycles defines a leak constant;

10. The apparatus of Claim 9, wherein the cycle limit is defined by a cycle constant fraction of a maximum pressure differential.

11. The apparatus of Claim 1, wherein the controller includes a first proportional-integral-derivative (PID) (108) controller and a second PID controller (110), the first PID controller (108) being part of a first control loop over the valve output pressure, and the second PID controller (110), together with the first control loop, being part of a second control loop for minimizing error between the patient interface pressure and a set pressure.

12. The apparatus of Claim 1, wherein the gas delivery conduit (30) and the pressure sensor line (38) are integrally formed; or
wherein the gas delivery conduit (30) and the pressure sensor line (38) are separated.

13. The apparatus of Claim 1, wherein the oxygen source (18, 18a, 18b) is a tank (18a) containing the pressurized oxygen enriched gas; or
wherein the oxygen source (18, 18a, 18b) is an oxygen concentrator in pneumatic communication with a compressor and wherein the oxygen concentrator outputs oxygen enriched gas at a first pressure level to the compressor; and
the compressor includes a pump having an input pneumatically coupled to the oxygen concentrator and an output pneumatically coupled to an accumulator which outputs the pressurized oxygen enriched gas at a second pressure level different from the first pressure level.

## Patentansprüche

1. Beatmungsvorrichtung, die umfasst:
einen Einlassanschluss (16), der mit einer Sauerstoffquelle (18, 18a, 18b) mit mit Sauerstoff angereichertem Druckgas verbunden werden kann;
einen Auslassanschluss (28), der über eine Gasabgabeleitung (30) mit einer Patientenschnittstelle (12) verbunden werden kann, die für die Anpassung an einen Patientenatemweg konfiguriert ist;
ein Ventil (26) mit einem Eingang in Druckluftverbindung mit dem Einlassanschluss (16) und mit einem Ausgang in Druckluftverbindung mit dem Auslassanschluss (28);
einen ersten Drucksensor (36), der einen Patientenschnittstellendruck misst, wobei die Patientenschnittstelle (12) über eine Drucksensorleitung (38) mit dem ersten Drucksensor (36) verbunden werden kann;
einen zweiten Drucksensor (34), der einen Ventilausgangsdruck misst; und
einen Controller in Verbindung mit dem ersten Drucksensor (36) und mit dem zweiten Drucksensor (34), **dadurch gekennzeichnet, dass** der Controller einen Durchschnitt einer Druckdifferenz ΔP zwischen dem Ventilausgangsdruck und dem Patientenschnittstellendruck berechnet und auf der Grundlage eines Vergleichs zwischen der Druckdifferenz ΔP und dem Durchschnitt eine Patienteneinatmungsphase detektiert.

2. Beatmungsvorrichtung gemäß Anspruch 1, wobei ein Durchmesser des Ventils (26) in einer vollständig geöffneten Stellung größer als ein Durchmesser der Gasabgabeleitung (30) ist.

3. Vorrichtung gemäß Anspruch 1, die ferner umfasst:
einen Durchflusssensor (43), der einen Gasdurchfluss misst und der mit dem Ausgang des Ventils (26) und mit dem Auslassanschluss (28) hintereinander angeordnet ist;
wobei der Controller auf der Grundlage einer Kombination von Messwerten des ersten Drucksensors (36), des zweiten Drucksensors (34) und des Durchflusssensors (43) die Patienteneinatmungsphase und die Patientenausatmungsphase detektiert.

4. Vorrichtung gemäß Anspruch 3, wobei der Controller die Abgabe des mit Sauerstoff angereicherten Druckgases an die Patientenschnittstelle (12) in Übereinstimmung mit einem Auslösegrenzwert und mit einem durch den Gasdurchfluss erreichten Zyklusgrenzwert regelt; wobei vorzugsweise
ein durchschnittlicher Gasdurchfluss über einen oder mehrere Patientenatmungszyklen eine Leckkonstante definiert; wobei optional
der Auslösegrenzwert durch die Leckkonstante, addiert mit einer Auslösekonstante, definiert ist.

5. Vorrichtung gemäß Anspruch 4, wobei der Zyklusgrenzwert durch einen Zykluskonstantenbruchteil eines maximalen Durchflusses definiert ist.

6. Vorrichtung gemäß Anspruch 3, wobei der Controller das Ventil (26) öffnet, um eine Strömung des mit Sauerstoff angereicherten Druckgases zu der Patientenschnittstelle (12) zu erzeugen, und das Ventil (26) schließt, um die Strömung zu verringern.

7. Vorrichtung gemäß Anspruch 3, wobei der Controller einen ersten Proportional-Integral-Differential-Controller (PID-Controller) (108) und einen zweiten PID-Controller (110) enthält, wobei der erste PID-Controller (108) Teil einer ersten Regelschleife über den Gasdurchfluss ist und wobei der zweite PID-Controller (110) zusammen mit der ersten Regelschleife Teil einer zweiten Regelschleife zum Minimieren des Fehlers zwischen dem Patientenschnittstellendruck und einem Solldruck ist.

8. Vorrichtung gemäß Anspruch 1, wobei der Controller das Ventil (26) öffnet, um eine Druckdifferenz bei der Patientenbeatmungsschnittstelle (12) zu erzeugen, und das Ventil (26) schließt, um die Druckdifferenz zu verringern.

9. Vorrichtung gemäß Anspruch 1, wobei der Controller die Abgabe des mit Sauerstoff angereicherten Druckgases an die Patientenschnittstelle (12) in Übereinstimmung mit einem Auslösegrenzwert und damit, dass durch die Druckdifferenzen zwischen dem Patientenschnittstellendruck und dem Ventilausgangsdruck ein Zyklusgrenzwert erreicht wird, regelt; wobei vorzugsweise eine durchschnittliche Druckdifferenz über einen oder mehrere Patientenatmungszyklen eine Leckkonstante definiert.

10. Vorrichtung gemäß Anspruch 9, wobei der Zyklusgrenzwert über einen Zykluskonstantenbruchteil einer maximalen Druckdifferenz definiert ist.

11. Vorrichtung gemäß Anspruch 1, wobei der Controller einen ersten Proportional-Integral-Differential-Controller (PID-Controller) (108) und einen zweiten PID-Controller (110) enthält, wobei der erste PID-Controller (108) Teil einer ersten Regelschleife über den Ventilausgangsdruck ist und wobei der zweite PID-Controller (110) zusammen mit der ersten Regelschleife Teil einer zweiten Regelschleife zum Minimieren des Fehlers zwischen dem Patientenschnittstellendruck und einem Solldruck ist.

12. Vorrichtung gemäß Anspruch 1, wobei die Gasabgabeleitung (30) und die Drucksensorleitung (38) einteilig gebildet sind; oder
wobei die Gasabgabeleitung (30) und die Drucksensorleitung (38) getrennt sind.

13. Vorrichtung gemäß Anspruch 1, wobei die Sauerstoffquelle (18, 18a, 18b) ein Behälter (18a) ist, der mit Sauerstoff angereichertes Druckgas enthält; oder
wobei die Sauerstoffquelle (18, 18a, 18b) ein Sauerstoffkonzentrator in Druckluftverbindung mit einem Kompressor ist und wobei
der Sauerstoffkonzentrator an den Kompressor mit Sauerstoff angereichertes Gas mit einem ersten Druckpegel abgibt; und
der Kompressor eine Pumpe enthält, die einen Eingang, der mit dem Sauerstoffkonzentrator pneumatisch gekoppelt ist, und einen Ausgang, der mit einem Druckspeicher, der das mit Sauerstoff angereicherte Druckgas mit einem zweiten Druckpegel, der von dem ersten Druckpegel verschieden ist, abgibt, pneumatisch gekoppelt ist, aufweist.

## Revendications

1. Dispositif de ventilation comprenant :
un orifice d'entrée (16) pouvant être connecté à une source d'oxygène (18, 18a, 18b) avec un gaz enrichi en oxygène sous pression ;
un orifice de sortie (28) pouvant être connecté sur un conduit de distribution de gaz (30) à une interface du patient (12) configurée pour l'adaptation à une voie respiratoire du patient ;
une soupape (26) avec une entrée en communication pneumatique avec l'orifice d'entrée (16) et une sortie en communication pneumatique avec l'orifice de sortie (28) ;
un premier capteur de pression (36) mesurant une pression d'interface du patient, l'interface du patient (12) pouvant être connectée au premier capteur de pression (36) sur une ligne de capteur de pression (38) ;
un deuxième capteur de pression (34) mesurant une pression de sortie de soupape ; et
un contrôleur en communication avec le premier capteur de pression (36) et le deuxième capteur de pression (34) **caractérisé en ce que** le contrôleur calcule une moyenne d'une différence de pression ΔP entre la pression de sortie de soupape et la pression d'interface du patient et détecte une phase inspiratoire du patient sur la base d'une comparaison entre la différence de pression ΔP et la moyenne.

2. Dispositif de ventilation selon la revendication 1, dans lequel un diamètre de la soupape (26) dans une position complètement ouverte est supérieur à un diamètre du conduit de distribution de gaz (30).

3. Dispositif selon la revendication 1, comprenant en outre :
un capteur de débit (43) mesurant un débit de gaz et en ligne avec la sortie de la soupape (26) et l'orifice de sortie (28) ;
dans lequel le contrôleur détecte la phase inspiratoire du patient et la phase expiratoire du patient sur la base d'une combinaison de mesures du premier capteur de pression (36), du deuxième capteur de pression (34) et du capteur de débit (43).

4. Dispositif selon la revendication 3, dans lequel le contrôleur régule la fourniture du gaz enrichi en oxygène sous pression à l'interface du patient (12) en fonction d'une limite de déclenchement et d'une limite de cycle atteintes par le débit de gaz ; de préférence dans lequel un débit de gaz moyen sur un ou plusieurs cycles respiratoires du patient définit une constante de fuite ; éventuellement
dans lequel la limite de déclenchement est définie par la constante de fuite ajoutée à une constante de déclenchement.

5. Dispositif selon la revendication 4, dans lequel la limite de cycle est définie par une fraction de constante de cycle d'un débit maximal.

6. Dispositif selon la revendication 3, dans lequel le contrôleur ouvre la soupape (26) pour induire un écoulement du gaz enrichi en oxygène sous pression vers l'interface du patient (12) et ferme la soupape (26) pour réduire l'écoulement.

7. Dispositif selon la revendication 3, dans lequel le contrôleur comprend un premier régulateur proportionnel-intégral-dérivé (PID) (108) et un deuxième régulateur proportionnel-intégral-dérivé (PID) (110), le premier régulateur PID (108) faisant partie d'une première boucle de régulation sur le débit de gaz et le deuxième régulateur PID (110), conjointement à la première boucle de régulation, faisant partie d'une deuxième boucle de régulation pour minimiser une erreur entre la pression d'interface du patient et une pression de consigne.

8. Dispositif selon la revendication 1, dans lequel le contrôleur ouvre la soupape (26) pour induire un différentiel de pression au niveau de l'interface de ventilation du patient (12) et ferme la soupape (26) pour réduire la différence de pression.

9. Dispositif selon la revendication 1, dans lequel le contrôleur régule la fourniture du gaz enrichi en oxygène sous pression à l'interface du patient (12) en fonction d'une limite de déclenchement et d'une limite de cycle atteintes par les différences de pression entre la pression d'interface du patient et la pression de sortie de soupape ; de préférence dans lequel une différence de pression moyenne sur un ou plusieurs cycles respiratoires du patient définit une constante de fuite.

10. Dispositif selon la revendication 9, dans lequel la limite de cycle est définie par une fraction de constante de cycle d'une différence de pression maximale.

11. Dispositif selon la revendication 1, dans lequel le contrôleur comprend un premier régulateur proportionnel-intégral-dérivé (PID) (108) et un deuxième régulateur PID (110), le premier régulateur PID (108) faisant partie d'une première boucle de régulation sur la pression de sortie de soupape et le deuxième régulateur PID (110), conjointement à la première boucle de régulation, faisant partie d'une deuxième boucle de régulation pour minimiser une erreur entre la pression d'interface du patient et une pression de consigne.

12. Dispositif selon la revendication 1, dans lequel le conduit de distribution de gaz (30) et la ligne de capteur de pression (38) sont formés de manière intégrée ; ou
dans lequel le conduit de distribution de gaz (30) et la ligne de capteur de pression (38) sont séparés.

13. Dispositif selon la revendication 1, dans lequel la source d'oxygène (18, 18a, 18b) est un réservoir (18a) contenant le gaz enrichi en oxygène sous pression ; ou
dans lequel la source d'oxygène (18, 18a, 18b) est un concentrateur d'oxygène en communication pneumatique avec un compresseur et dans lequel le concentrateur d'oxygène délivre en sortie du gaz enrichi en oxygène à un premier niveau de pression au compresseur ; et
le compresseur comprend une pompe ayant une entrée couplée de manière pneumatique à un concentrateur d'oxygène et une sortie couplée de manière pneumatique à un accumulateur qui délivre en sortie le gaz enrichi en oxygène sous pression à un deuxième niveau de pression différent du premier niveau de pression.
